# EUROPÄISCHE PATENTANMELDUNG

(11) **EP 4 144 898 A1**
(43) Veröffentlichungstag der Anmeldung: **08.03.2023**
(21) Anmeldenummer: 21195411.0
(22) Anmeldetag: 07.09.2021
(51) Int. Cl.: C40B 30/06, C40B 40/02, G01N 33/68, C07K 16/00

(54) **VERFAHREN ZUR SELEKTION ODER SCREENEN VON ANTIKÖRPERN AUS EINER ANTIKÖRPERBIBLIOTHEK**

(71) Anmelder: New/era/mabs GmbH, 14482 Potsdam (DE)
(72) Erfinder: HANACK,Katja, 14169 Berlin (DE); LISTEK, Martin, 13059 Berlin (DE); SCHLÖR, Anja, 14467 Potsdam (DE)
(74) Vertreter: Simandi, Claus

(57) **Zusammenfassung**

Die Erfindung betrifft ein neues Verfahren zur Selektion oder zum Screenen von Antikörpern und Antigenen aus einer Antikörperbibliothek samt geeigneten Expressionsvektoren und deren Verwendung.

## Beschreibung

Die Erfindung betrifft ein neues Verfahren zur Selektion oder zum Screenen von Antikörpern und Antigenen aus einer Antikörperbibliothek samt geeigneten Expressionsvektoren und deren Verwendung.

Monoklonale Antikörper werden in der Regel mit Hilfe der Hybridomtechnik gewonnen, wobei antikörperproduzierende Zellen (B-Zellen oder B-Lymphozyten) mit Myelomzellen (Krebszellen) fusionieren, auch mittels Fusionszelllinien, woraufhin Hybride entstehen, die monoklonale Antikörper produzieren (G. Köhler, C. Milstein: Continuous cultures of fused cells secreting antibody of predefined specificity. In: Nature. Band 256, 1975, S. 495-497). Die Identifizierung der Hybridomzelle, die einen gegen das Antigen gerichteten Antikörper produziert, kann besonders vorteilhaft mit der SELMA^{®} Technologie der Anmelderin erfolgen, wobei ein aus der Hybridomzelle erfolgreich sezernierter Antikörper (Ak) von dieser Hybridomzelle über eine Ak-Fängermatrix oder einem Antigen eingefangen bzw. an der Zelloberfläche gebunden wird und z.B. mittels fluoreszenzmarkierter Nachweis-Antikörper identifiziert wird.

Die SELMA^{®} Technologie wird in WO2015161835 beschrieben. Insbesondere werden durch die Verwendung artifizieller Oberflächenmarker die Antikörper-produzierenden Hybridomzellen modifiziert. Dadurch entsteht eine direkte Verbindung zwischen dem Antikörperphänotyp und dem Genotyp der Hybridomzelle. Auf diese Weise können Hybridomzellen, die die gewünschten Antikörper produzieren mittels MACS- oder FACS-Technik äußerst effizient, sowie zeitsparend angereichert werden.

WO 2015161835 A1 beschreibt zudem den Einsatz einer Ligationspeptidsequenz, mit der Funktion, dass die Bindung eines Adapterliganden erfolgen kann, wie z.B. Avidin/Biotin. An den Adapterliganden erfolgt die Bindung des freigesetzten Antikörpers aus der Hybridomzelle über einen Epitop aufweisenden Antigen-(Strept)avidin-Komplex, wobei der Antikörper an das Epitop des Antigens bindet und über die (Strept)avidin-Biotin Bindung (Adapterliganden), wiederum der Hybridomzelle zugeordnet und (aus)sortiert werden kann. Das Antigen kann alternativ mit einer Antikörper-Fängermatrix ersetzt werden.

Mit Hilfe der Durchflusszytometrie können beispielsweise solche Fluoreszenz-markierte Konstrukte mit einem flow sorter (Fluss-Sortierer) oder FACS (fluorescence-activated cell sorting) sortiert und isoliert werden.

WO 2015161835 A1 offenbart jedoch keine Antikörperbibliotheken und eine Selektion von Antikörpern daraus. Weiterhin wird in WO 2015161835 A1 nicht das Screenen von Antikörpern mittels eines oder mehreren Antigenen offenbart.

Antikörper können im Stand der Technik mit Phage-Display-Verfahren erzeugt werden (z.B. WO91/17271, WO92/001047, WO92/20791) oder aus einer humanen kombinatorischen monoklonalen Antikörperbibliothek ausgewählt werden (z.B. HuCAL^{®} von Morphosys^{®}). Humane Antikörper können auch unter Verwendung von transgenen Tieren hergestellt werden, die ein menschliches Immunglobulin-Gen tragen (z.B. WO93/12227). Es wurden große Bibliotheken von vollständig oder teilweise synthetischen Antikörperverbindungsstellen oder Paratopen unter Verwendung filamentöser Phagen-Display-Vektoren hergestellt, die als Phagemide bezeichnet werden, welche zu großen Bibliotheken monoklonaler Antikörper mit verschiedenen und neuen Immunspezifitäten führte. Die Technologie verwendet eine filamentöse Phagenmantelproteinmembranankerdomäne als Mittel zur Verbindung des Genprodukts und dem Gen, während dem Stadium des Zusammenbaus der filamentösen Phagenreplikation, und wurde zur Klonierung und Expression von Antikörpern aus kombinatorischen Bibliotheken verwendet.

Zum Beispiel kann ein Phage Display, wie folgt bereitgestellt werden:
Phagen-Display Antikörperproduzierende B-Zellen oder deren mRNA werden z.B. aus Humanblut isoliert. Nach der Herstellung einer cDNA werden mittels PCR (Polymerase-Chain-Reaction) die DNA-Fragmente, die für die variablen Regionen (V_{L}, V_{H}) der Gene der leichten und schweren Ketten kodieren, vervielfältigt. In einer zweiten PCR werden V_{L} - und V_{H} - Fragmente über eine Linker-Sequenz, die für das scFv-Fragment kodiert, miteinander verbunden. Die entstandenen Fragmente kodieren für V_{H} -G-G-G-G-S-S-S-V_{L} - Sequenzen ("AK"). Die PCR-Fragmente können beispielweise unter Verwendung von Phagemid-Vektoren an ein DNA-Fragment, das aus einem verkürzten, für das Hüllprotein pIII (minor coat protein) des M13-Phagen kodierenden Gen und einem für ein Signalpeptid kodierenden Gen besteht, gebunden und in E. coli exprimiert werden. Das Signalpeptid bewirkt, dass die Fusionsproteine in das Periplasma gelangen. Nach Koinfektion mit einem M13-Helferphagen, der die Bildung des nicht modifizierten pIII und anderer Phagenproteine erlaubt, werden die fusionierten "AK" in die Außenhülle des Phagen integriert. Die Phagen enthalten die genetische Information des jeweiligen "AK"-Proteins, das sie auf ihrer Oberfläche präsentieren. Die Methode, mit der die Phagen, die das "richtige" "AK"-Protein präsentieren, über seine Antigenbindefähigkeiten erkannt werden können, wird "Biopanning" genannt. Auf diese Weise werden die DNA-Sequenz der DNA-Fragmente erhalten, die für die variablen Regionen der humanen leichten und humanen schweren Ketten eines ein bestimmtes Antigen bindenden AK kodieren, welche nachfolgend mit DNA verbunden werden, die für einen humanen Fc-Teil eines "AK" kodieren, um ein komplettes für einen "AK" kodierendes Gen zu erhalten. Das Verfahren ist jedoch aufwändig und zeitintensiv. Alternative Displays wie Ribosom Display sind im Stand der Technik beschrieben.

Solche Antikörperbibliotheken sind Gegenstand der vorliegenden Erfindung. Eine Antikörperbibliothek kann auf ein Target, insbesondere Antigen gescreent und die Binder werden über einen Enzym-gekoppelten Immunoassay selektiert. Anschließend müssen die Antikörpergene isoliert und umkloniert werden, um das identifizierte Antikörper-Fragment zu produzieren. Da der Phagendisplay zumeist den variablen Teil des Antikörpers aufweist, muss für die Produktion eines Volllängenpeptids noch ein geeigneter Fc Teil angefügt werden. Die Nachteile dieser Lösung liegen in den umfangreichen Arbeitsschritten, wie z.B. ein erheblicher Klonierungsaufwand für die Überführung von geeigneten Antikörper-Kandidaten in die Antikörper-Produktion. Jeder Antikörperkandidat muss einzeln kloniert werden. Sollten die Antikörper nach Produktion und Reinigung nicht funktional sein, muss der gesamte Prozess inklusive Phagendisplay wiederholt werden.

Daher besteht die Aufgabe das aufwändige Screeningverfahren zu verbessern.

Überraschenderweise kann durch die Integration einer Antikörperbibliothek in einen Expressionsvektor zur Selektion von gescreenten Antikörpern mittels eines Antigens direkt eine Produktion, insbesondere eines Volllängenformats durchgeführt werden, da der identifizierte Antikörper (oder Teile davon) unmittelbar auf die Produktionszelle hinweist. Weiterhin ist überraschend, dass der Einsatz von mehreren Antigenen zugleich möglich ist, und dennoch ein Screening erfolgen kann, obwohl durch die Sezernierung der verschiedenen Antikörper in das Kulturmedium eine (Fremd-)Beladung durch unspezifische Antigene erfolgen kann. Dies gilt ebenfalls für das erfindungsgemäße Oberflächenprotein enthaltend eine Ligationspeptidsequenz. Besonders überraschend ist daher, dass das erfindungsgemäße Verfahren für unterschiedliche sezernierte Antikörper aus sogar verschiedenen Antikörperbibliotheken erfolgreich simultan durchgeführt werden kann.

Die Aufgabe wird daher durch die technische Lehre von mindestens einem Patentanspruch gelöst.

Daher betrifft die Erfindung ein Verfahren zur Selektion oder zum Screenen ein oder mehrerer Antikörper aus ein oder mehreren Antikörperbibliotheken, wobei mindestens einem nach seiner Transformation stabil in das Genom integrierte Polynukleotide in einem Expressionsvektor in einer Wirtszelle verwendet wird, wobei der Expressionsvektor mindestens aufweist:
i.) Polynukleotide kodierend für mindestens eine Antikörperbibliothek,
ii.) Polynukleotide kodierend für ein Oberflächenprotein enthaltend eine Ligationspeptidsequenz und / oder mindestens ein Epitop für mindestens einen Antikörper aus einer oder mehreren Antikörperbibliotheken,
wobei
a.) die Antikörper der Antikörperbibliothek aus der Wirtszelle in das Kulturmedium sezernieren und an mindestens ein zugegebenes Antigen binden, welche einen Adapter aufweist, wobei der Adapter an die Ligationspeptidsequenz aus ii.) bindet,
   und /oder
b.) die Antikörper der Antikörperbibliothek aus der Wirtszelle in das Kulturmedium sezernieren und an mindestens ein zugegebenes Antigen binden, wobei der sezernierte Antikörper an mindestens ein Epitop aus ii.) bindet.

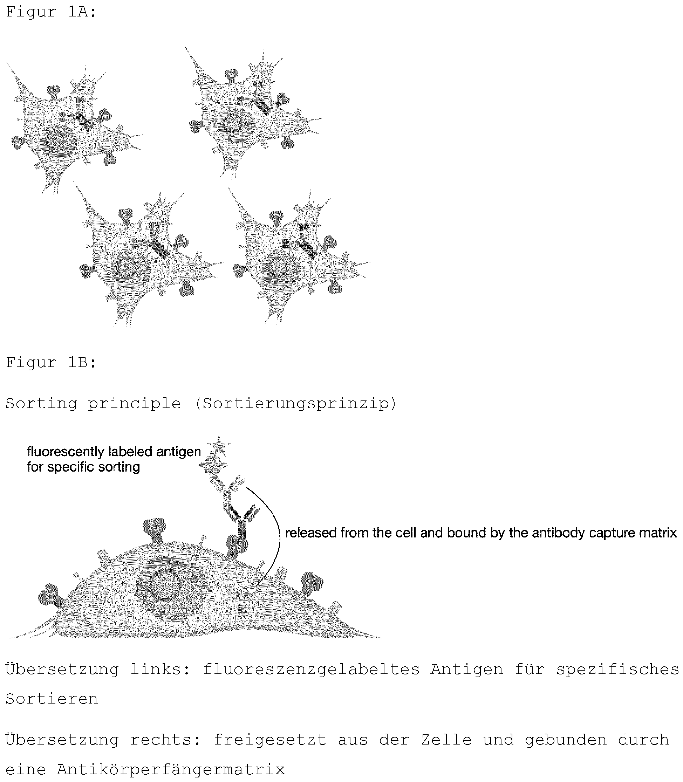

Eine solche erfindungsgemäße Ausgangssituation an bereitgestellten Antikörperbibliotheken ist in Figur 1A dargestellt.

Beispielweise kann der Fc-Teil des sezernierten Antikörpers an ein Epitop aus b.) binden.

In einer weiteren bevorzugten Ausführungsform kann eine Markierung des gebundenen Antigens und /oder des gebundenen Antiköpers aus a.) oder b.) erfolgen, und zwar nach üblichen Methoden beispielsweise mit gelabelten (Sekundär-)Antikörpern (z.B. Fluoreszenz) oder magnetischen Beads.

Mit Hilfe der Durchflusszytometrie können beispielsweise solche Fluoreszenz-markierte Konstrukte gemäß Figur 1B (Antikörper-Fängermatrix) oder einem Antigen mit einem flow sorter (Fluss-Sortierer) oder FACS (fluorescence-activated cell sorting) sortiert und isoliert werden.

Mithilfe des mindestens einen zugegebenen Antigens kann nach einem geeigneten Antikörper aus der Antikörperbibliothek gescreent, und der Antikörper z.B. nach Zellsortierung produziert werden.

Daher betrifft die Erfindung die Verwendung von mindestens einem oder mehr als einem Antigen zur Selektion oder zum Screenen ein oder mehrerer Antikörper aus ein oder mehreren Antikörperbibliotheken, wobei mindestens einem nach seiner Transformation stabil in das Genom integrierte Polynukleotide in einem Expressionsvektor in einer Wirtszelle verwendet wird, wobei der Expressionsvektor mindestens aufweist:
i.) Polynukleotide kodierend für mindestens eine Antikörperbibliothek,
ii.) Polynukleotide kodierend für ein Oberflächenprotein enthaltend eine Ligationspeptidsequenz und / oder mindestens ein Epitop für mindestens einen Antikörper aus einer oder mehreren Antikörperbibliotheken,
wobei
a.) die Antikörper der Antikörperbibliothek aus der Wirtszelle in das Kulturmedium sezernieren und an mindestens ein zugegebenes Antigen binden, welche einen Adapter aufweist, wobei der Adapter an die Ligationspeptidsequenz aus ii.) bindet,
   und /oder
b.) die Antikörper der Antikörperbibliothek aus der Wirtszelle in das Kulturmedium sezernieren und an mindestens ein zugegebenes Antigen binden, wobei der sezernierte Antikörper an mindestens ein Epitop aus ii.) bindet.

Das "mindestens eine Antigen" kann beliebiger Natur sein und insbesondere eine chemische Substanz, Protein, Peptid, Wirkstoff, Medikament oder ein Antiköper sein. "Mindestens ein Antigen" heißt, dass die Antigene gleich oder vorzugsweise verschieden sind, und zwar hinsichtlich der chemischen Zusammensetzung und der jeweiligen Menge bzw. Konzentration. Beispielsweise können mehr als ein Antigen, insbesondere 100 oder 1000 oder mehr verschiedene Antigene eingesetzt werden.

Überraschender Weise ist das erfindungsgemäße Verfahren hinreichend robust, auch mit dieser Vielzahl an Antikörpern und Antigenen zurecht zu kommen und eine Selektion bzw. Auswahl (Screenen) von Antikörpern zu Antigenen und vice versa zu ermöglichen.

Das besagte Epitop ist Gegenstand des Oberflächenproteins, insbesondere kann das Epitop als Teil eines Antikörpers oder eines Antigens ausgebildet sein.

Die Wirtszelle ist besonders bevorzugt eine Säugetierzelle einschließlich Humanzelle, insbesondere ausgewählt aus HEK293-Zellen, CHO-Zellen, insbesondere Zelllinien, solche wie, nicht abschließend, NS0, Sp2/0, PER.C6, BHK, COS-7 u.a.. Geeignete Zellen zur Antikörperproduktion sind dem Fachmann bekannt (Kunert R, Reinhart D. Advances in recombinant antibody manufacturing. Appl Microbiol Biotechnol. 2016 Apr;100(8):3451-61. doi: 10.1007/s00253-016-7388-9). Die Wirtszelle ist jedoch keine Hybridomzelle.

Die besagte Ligationspeptidsequenz weist im Fall von Biotin eine Biotinakzeptorpeptidsequenz bzw. Biotinylisierungspeptid, wie vorzugsweise GLNDIFEAQKIEWHE (SEQ ID No. 1) oder LNDIFEAQKIEWH (SEQ ID No. 2) auf. Auch andere Biotinakzeptorpeptidsequenz bzw. Biotinylisierungspeptide können geeignet sein, wie ein Alignment mit 13 Aminosäuren 1-LXXIXXXXKX XXX-13 gemäß SEQ ID. No. 3 (vgl. D. Beckett, E. Kovaleva, and P. J. Schatz, A minimal peptide substrate in biotin holoenzyme synthetase-catalyzed biotinylation, Protein Sci. 1999 Apr; 8(4): 921-929), wobei:
Aminosäure 2 N, C oder beliebig oder entfernt sein kann,
Aminosäure 3 beliebig, jedoch ausgenommen L, V, I, W, F, Y sein kann,
Aminosäure 5 F oder L sein kann,
Aminosäure 6 E oder D sein kann,
Aminosäure 7 A, G, S, T sein kann,
Aminosäure 8 Q oder M sein kann,
Aminosäure 10 I, M, V sein kann,
Aminosäure 11 E, L, V, Y, I sein kann,
Aminosäure 12 W, Y, V, F, L, I sein kann,
Aminosäure 13 R, H oder beliebig, jedoch ausgenommen D, E.

### Abb. 1: Biotinylierung von AviTag (Biotinylisierungspeptid) mittels BirA (Biotin-Proteinligase)

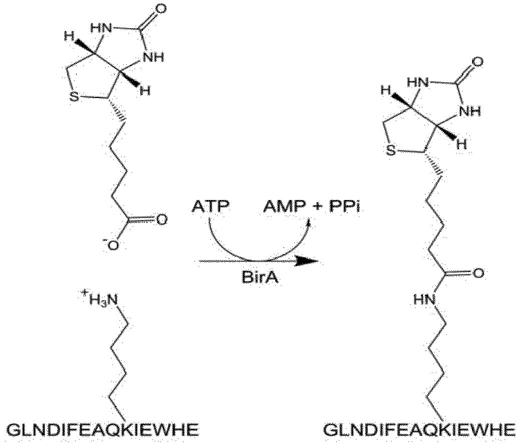

In einer bevorzugten Ausführungsform der Erfindung weist der Expressionsvektor Polynukleotide kodierend für mindestens eine Biotin-Proteinligase (BirA) auf. Besonders vorteilhaft wird erfindungsgemäß Biotin-Proteinligase (BirA) intrazellulär freigesetzt. Die Biotinylierung eines Biotinylierungspeptid erfolgt intrazellulär vorzugsweise im endoplasmatischen Retikulum. Anschließend wird der biotinylierte Rezeptor bzw. das Biotin aufweisende Biotinylisierungspeptid an die Zelloberfläche transportiert, und zwar in Form eines Oberflächenproteins aufweisend ein Biotinylierungspeptid, welches Biotin bindet und präsentiert. In einer weiteren bevorzugten Ausführungsform enthält BirA ein Retentionssignal für das endoplasmatische Retikulum, wie KDEL (SEQ ID No. 4).

Das zugegebene Antigen weist einen Adapter auf, wobei der Adapter an das Biotin des Biotinylisierungspeptids bindet, und das gebundene Antigen markiert wird und der Adapter ist ausgewählt aus Avidin, Streptavidin, Neutravidin.

Insbesondere erfolgt das erfindungsgemäße Verfahren unter natürlichen, physiologischen Bedingungen (z.B. Kulturmedium im Brutschrank) bei dem die Wirtszellen im Kulturmedium bleiben. Die Vitalität der Zellen bleibt vorteilhaft unberührt. Das Kulturmedium kann z.B. Biotin u.a. Hilfs- und Zusatzstoffe enthalten.

In einer weiteren bevorzugten Ausführungsform erfolgt die Zugabe der mehreren und verschiedenen Antigene in der Kälte, wie z.B. 4 Grad Celsius, sodass eine Sezernierung des Antikörpers nicht erfolgen kann. Die besagte Zugabe oder Inkubation kann vorzugsweise sukzessiv erfolgen, beispielsweise in Intervallen von 20 - 40 Minuten, sodass z.B. nach Entnahme der Wirtszellen eine erneute Zugabe (bzw. Inkubation) erfolgen kann bzw. die Antigene erneut im Kulturmedium suspendieren können.

Anschließend können in der Wärme, wie z.B. 37 Grad Celsius die Antikörper der Antikörperbibliothek aus der Wirtszelle in das Kulturmedium sezernieren.

Daher betrifft die Erfindung ein Verfahren, wobei die Zugabe der mehreren und verschiedenen Antigene in der Kälte erfolgt und anschließend in der Wärme die Antikörper der Antikörperbibliothek aus der Wirtszelle in das Kulturmedium sezernieren.

In einem weiteren vorteilhaften Verfahrensschritt können zur Vermeidung von unspezifischen Bindungen Blockierungsmittel, wie freies Avidin, Streptavidin, Neutravidin an Biotin des Biotinylisierungspeptids eingesetzt werden.

Daher betrifft die Erfindung ein Verfahren, wobei zusätzlich Blockierungsmittel nach Zugabe des mindestens einen Antigens in das Kulturmedium hinzugegeben werden.

In einer bevorzugten Ausführungsform enthält der erfindungsgemäße Expressionsvektor folgende weitere Merkmale, wie:
Signalsequenz, HA-Tag (SEQ ID No. 5, SEQ ID No. 6), DNA-Biotinylierungssequenz (SEQ ID No. 7), EGFR-Signalsequenz (z.B. SEQ ID No. 8, SEQ ID No. 9), EGFR-Sequenz (SEQ ID No. 10, SEQ ID No. 11 inkl. Transmenbrandomäne), IRES (SEQ ID No. 12) als auch birA-Sequenz (SEQ ID No. 13 inkl. Retentionssignal).

Die angegebenen Sequenzen umfassen sowohl Polynukleotide als auch die exprimierten und translatierten Sequenzen.

Bevorzugte geeignete Promotoren sind nicht abschließend EF-1, SV-40, CMV, CAG, u.v.a. auch konditionierbare Promotoren, wie TET on, off, lichtabhängige Promotoren oder stressinduzierbare Promotoren (z.B. Temperatur) als auch mit Hilfe der Verwendung von Hilfssequenzen, wie IRES (internal ribosome entry site) 2A Peptide u.a.. Solche Promotoren und Hilfssequenzen sind dem Fachmann einschlägig bekannt.

Das erfindungsgemäße Oberflächenprotein kann beispielsweise mit Hilfe von einem Epidermal Growth Factor Receptor (EGFR (supra), ErbB-1, HER1 u.a.) sicher an die Zelloberfläche transportiert und an der Zelloberfläche repräsentiert werden. Weiterhin kann das Oberflächenprotein ein Kontrollepitop, wie z.B. Hämagglutinin-A-Epitop YPYDVPDYA (SEQ ID No. 14) aufweisen, so dass beispielsweise cytometrisch oder fluoreszenzmikroskopisch mit Hilfe eines Antikörpers festgestellt werden kann, ob das Oberflächenprotein an der Zellmembran extrazellulär präsent ist.

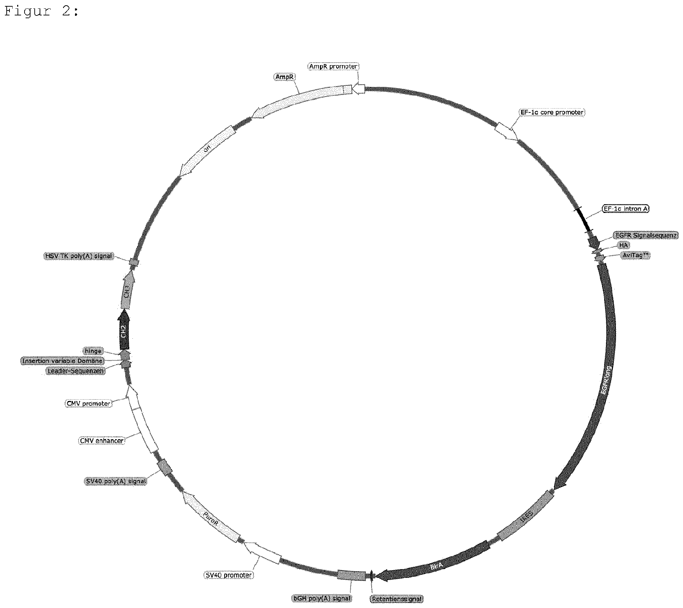

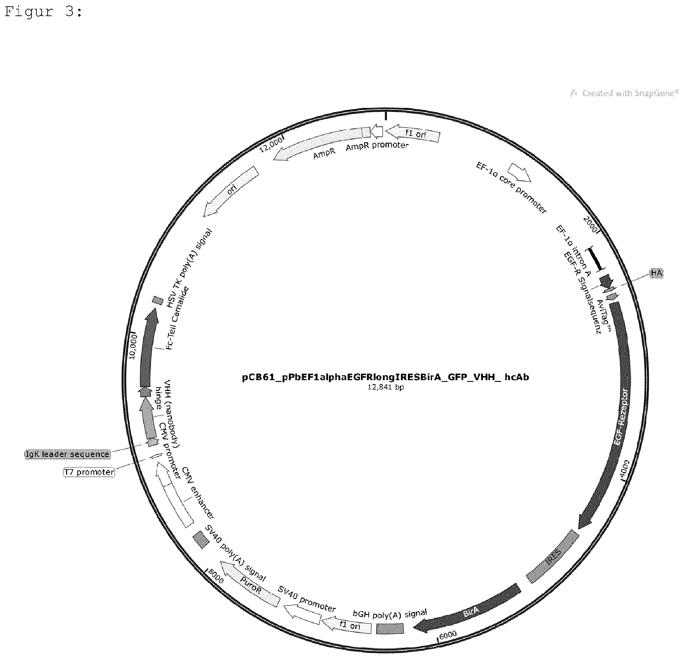

Ein erfindungsgemäßer Expressionsvektor ist in seinen funktionellen Einheiten (Vektorkarte) beispielhaft in den Figuren 2 und 3 wiedergegeben.

Ein konkret geeigneter Expressionsvektor ist beispielhaft in Figur 3 wiedergegeben (SEQ ID No. 15).

Selbstredend kann der Expressionsvektor in zwei oder mehr Einheiten aufgeteilt werden. Der Fachmann ist in der Lage entsprechende Expressionsvektoren herzustellen und diese vorzugsweise gemeinsam - simultan oder nacheinander - in die Wirtszelle, vorzugsweise Säugetierzelle oder Humanzelle einzubringen.

In einer weiteren Ausführungsform kann ebenfalls ein duales System bestehend aus einem Donor- und Helferplasmid verwendet werden, bei dem die Transposase auf einem Helferplasmid kodiert ist. Das Donorplasmid beinhaltet eine Kassette, die ins Genom integriert wird. Es besteht auch die Möglichkeit, dass die Transposase und das Donor-Konstrukt auf einem Vektor kodiert sind.

Der Begriff "Antikörper" im Sinne dieser Erfindung betrifft Immunglobulinmoleküle oder immunologisch aktive Teile von Immunglobulinmolekülen, d. h., Moleküle, die eine antikörperverbindende Stelle oder ein Paratop enthalten.

Beispielhafte Antikörpermoleküle sind intakte Immunglobulinmoleküle, im Wesentlichen intakte Immunglobulinmoleküle und Teile eines Immunglobulinmoleküls, einschließlich der auf dem Fachgebiet als Fab, Fab', F(ab')2 und F(v) bezeichneten Teile.

Ein Antikörper ist daher ein Protein mit einem oder mehreren Polypeptiden, die von Immunglobulingenen bzw. Polynucleotiden kodiert sind, die spezifisch an mindestens einem Epitop ein Antigen binden. Zu den bekannten Immunglobulingenen gehören die Kappa-, Lambda-, Alpha- (IgA), Gamma- (IgG1, IgG2, IgG3, IgG4), Delta- (IgD), Epsilon- (IgE) und Mu-(IgM)- Gene der konstanten Region sowie die unzähligen Immunglobulingene der variablen Region. Leichte Immunglobulinketten der vollen Länge sind in der Regel ca. 25 kD oder 214 Aminosäuren lang. Schwere Immunglobulinketten der vollen Länge sind in der Regel ca. 50 kD oder 446 Aminosäuren lang. Leichte Ketten werden durch ein Gen der variablen Region am NH₂-Terminus (ca. 110 Aminosäuren lang) und durch ein Kappa- oder Lambda-Gen der konstanten Region am COOH-Terminus codiert. Schwere Ketten werden in ähnlicher Weise durch ein Gen der variablen Region (ca. 116 Aminosäuren lang) und durch ein Gen der anderen konstanten Region codiert.

Die grundlegende strukturelle Einheit eines Antikörpers ist in der Regel ein Tetramer, das aus zwei identischen Paaren von Immunglobulinketten besteht, wobei jedes Paar eine leichte und eine schwere Kette enthält. In jedem Paar binden die variablen Regionen ("variable domain") der leichten und der schweren Kette an ein Antigen und die konstanten Regionen ("constant domain") vermitteln Effektorfunktionen. Immunglobuline treten auch in einer Vielfalt von anderen Formen, darunter zum Beispiel Fv, Fab, und (Fab')2, sowie als bifunktionelle hybride Antikörper und Einzelketten auf. Eine variable Region einer leichten oder schweren Kette eines Immunglobulins umfasst eine Rahmenregion, die von drei hypervariablen Regionen, auch als Komplementarität bestimmende Regionen (Complementarity Determining Regions, CDR) bekannt, unterbrochen wird. Wie ausgeführt, sind die CDR in erster Linie verantwortlich für die Bindung an ein Epitop eines Antigens. Ein Immunkomplex ist ein Antikörper wie zum Beispiel ein monoklonaler Antikörper, chimärer Antikörper, humanisierter Antikörper oder humaner Antikörper oder ein funktionales Antikörperfragment, der spezifisch an das Antigen gebunden ist. Chimäre Antikörper sind Antikörper, deren leichte und schwere Kettengene aus Immunglobulingenen der variablen und konstanten Region aus verschiedenen Spezies hergestellt wurden (z.B. Maus Mensch u.a.), in der Regel durch gentechnische Verfahren.

Der Begriff "antikörperverbindende Stelle" umfasst den strukturellen Anteil eines Antikörpermoleküls, der aus variablen und hypervariablen Regionen von schweren und leichten Ketten besteht, die spezifisch ein Antigen binden, und folglich eine Immunreaktion beschreiben, wobei das sog. Paratop des Antikörpers an das sog. Epitop des Antigens bindet.

Der Begriff "Immunreaktion" bedeutet eine spezifische Bindung zwischen einem Molekül, das eine antigene Determinante enthält, und einem Molekül, das eine antikörperverbindende Stelle enthält, wie ein Gesamtantikörpermolekül oder ein Teil davon.

Ebenfalls erfindungsgemäß umfasst sind erfindungsgemäß kamelide Antikörper, welche keine leichten Ketten aufweisen und daher in der Lage sind, die Antigenbindung bzw. Immunreaktion mit nur einer Kette zu realisieren.

Weiterhin sind erfindungsgemäß solche Antikörper umfasst, die rekombinant hergestellt werden können, insbesondere monoklonale Antikörper, im Fall von kameliden Antikörper, sogenannte Nanobodies oder VHH bzw. VH Fragmente (Aline Desmyter, Silvia Spinelli, Alain Roussel, Christian Cambillau, Camelid nanobodies: killing two birds with one stone, Current Opinion in Structural Biology, Volume 32, 2015, Pages 1-8, ISSN 0959-440X, https://doi.org/10.1016/j.sbi.2015.01.001.).

Im Sinne dieser Erfindung ist eine Antikörperbibliothek eine solche, welche mindestens zwei und mehr verschiedene Antikörper bereitstellt, wobei zumindest im erfindungsgemäßen Expressionsvektor Polynukleotide kodierend für mindestens eine variable Region ("variable domain") der leichten und/oder der schweren Kette und mindestens eine konstante Region ("constant domain") aufweisen. Der Fachmann ist in der Lage solche Antikörperbibliotheken in einem Expressionsvektor bereitzustellen, wie vorstehend beschrieben. Bevorzugt weist die konstante Region ("constant domain") mindestens einen Fc-Teil auf, insbesondere mindestens einen humanen, murinen oder kameliden Fc. Antikörperbibliotheken sind z.B. beschreiben in: Roger R. Beerli, Monika Bauer, Regula B. Buser, Myriam Gwerder, Simone Muntwiler, Patrik Maurer, Philippe Saudan, Martin F. Bachmann, Isolation of human monoclonal antibodies by mammalian cell display, Proceedings of the National Academy of Sciences Sep 2008, 105 (38) 14336-14341; DOI: 10.1073/pnas.0805942105; Hoogenboom HR. Selecting and screening recombinant antibody libraries. Nat Biotechnol. 2005 Sep;23(9):1105-16. doi: 10.1038/nbt1126. PMID: 16151404; Ledsgaard L, Kilstrup M, Karatt-Vellatt A, McCafferty J, Laustsen AH. Basics of Antibody Phage Display Technology. Toxins (Basel). 2018 Jun 9;10(6):236. doi: 10.3390/toxins10060236. PMID: 29890762; PMCID: PMC6024766; Kumar R, Parray HA, Shrivastava T, Sinha S, Luthra K. Phage display antibody libraries: A robust approach for generation of recombinant human monoclonal antibodies. Int J Biol Macromol. 2019 Aug 15;135:907-918. doi: 10.1016/j.ijbiomac.2019.06.006. Epub 2019 Jun 3. PMID: 31170490; Reader RH, Workman RG, Maddison BC, Gough KC. Advances in the Production and Batch Reformatting of Phage Antibody Libraries. Mol Biotechnol. 2019 Nov;61(11):801-815. doi: 10.1007/s12033-019-00207-0. PMID: 31468301; PMCID: PMC6785589.

Ferner kann die erfindungsgemäße Antikörperbibliothek eine kombinatorische oder mutagenisierte Antikörperbibliothek umfassen, insbesondere naive, immune, nicht-immune und semisynthetische Antikörperbibliotheken (siehe: Lim CC, Choong YS, Lim TS. Cognizance of Molecular Methods for the Generation of Mutagenic Phage Display Antibody Libraries for Affinity Maturation. Int J Mol Sci. 2019 Apr 15;20(8):1861. doi: 10.3390/ijms20081861. PMID: 30991723; PMCID: PMC6515083; Ponsel D, Neugebauer J, Ladetzki-Baehs K, Tissot K. High affinity, developability and functional size: the holy grail of combinatorial antibody library generation. Molecules. 2011 May 3;16(5):3675-700. doi: 10.3390/molecules16053675; McConnell AD, Do M, Neben TY, Spasojevic V, MacLaren J, Chen AP, Altobell L 3rd, Macomber JL, Berkebile AD, Horlick RA, Bowers PM, King DJ. High affinity humanized antibodies without making hybridomas; immunization paired with mammalian cell display and in vitro somatic hypermutation, PLoS One. 2012;7(11):e49458. doi: 10.1371/journal.pone.0049458).

Zur Expression der variablen Regionen ("variable domain) und der konstanten Regionen ("constant domain") werden sogenannte Protein-Leader Sequenzen eingesetzt, insbesondere vorzugsweise solche wie, murines Ig heavy chain V region 102 (SEQ. ID No. 16), murine Ig Kappa (SEQ. ID No. 17), humanes Proinsulin (SEQ. ID No. 18), humanes Interleukin-2 (SEQ. ID No. 19) und humanes Trypsinogen-2 (Seq. ID No. 20)) (Marion E.E. Watson, Compilation of published signal sequences, Nucleic Acids Research, Volume 12, Issue 13, 11 July 1984, Pages 5145-5164, https://doi.org/10.1093/nar/12.13.5145, Owji H, Nezafat N, Negahdaripour M, Hajiebrahimi A, Ghasemi Y. A comprehensive review of signal peptides: Structure, roles, and applications. Eur J Cell Biol. 2018 Aug;97(6):422-441. doi: 10.1016/j.ejcb.2018.06.003, Stroud RM, Walter P. Signal sequence recognition and protein targeting. Curr Opin Struct Biol. 1999 Dec;9(6):754-9. doi: 10.1016/s0959-440x(99)00040-8, Chen Y, Shanmugam SK, Dalbey RE. The Principles of Protein Targeting and Transport Across Cell Membranes. Protein J. 2019 Jun;38(3):236-248. doi: 10.1007/s10930-019-09847-2.

Daher umfasst die Erfindung einen Expressionsvektor mit Polynukleotiden kodierend für eine Antikörperbibliothek mit mindestens einer variablen Region der leichten und/oder der schweren Kette und mindestens einer konstanten Region, insbesondere mindestens einen humanen, murinen oder kameliden Fc-Teil, und mindestens einer Protein-Leader Sequenz.

Weiterhin umfasst die Erfindung einen Expressionsvektor aufweisend
i.) Polynukleotide kodierend für mindestens eine Antikörperbibliothek,
ii.) Polynukleotide kodierend für ein Oberflächenprotein enthaltend eine Ligationspeptidsequenz und / oder mindestens ein Epitop für mindestens einen Antikörper aus einer oder mehreren Antikörperbibliotheken.

Weiterhin kann der Expressionsvektor weitere Ausführungsformen aufweisen, wie vorstehend beschrieben.

Ferner betrifft die Erfindung eine Säugetierzelle, die einen erfindungsgemäßen Expressionsvektor umfasst.

Die folgenden Beispiele und Figuren sollen die Erfindung näher erläutern, ohne jedoch diese zu beschränken.

### Beispiel 1:

### Zelllinie und Zellkultur

Humane embryonale Nierenzellen (Expi293F HEK) wurden bei 37°C, 125 rpm und 8% CO₂ in Expi293 Expression Medium (ThermoFisher; Waltham, USA) kultiviert.

### Beispiel 2:

Die Transfektion der Expi293F HEK Zelllinie (ThermoFisher; Waltham, USA) mit der Antikörperbibliothek und dem Oberflächenmarker-exprimierenden Genkonstrukt HA-AP-EGF-R (Fig. 2) wurde in einer dynamischen Kultur durchgeführt. Die Expi293F HEK Zellen wurden am Tag der Transfektion auf eine Zellzahl von 150E6 Zellen in 50 mL Expi293 Expression Medium eingestellt. Für die Transfektion der Expi293F HEK Zellen wurden 50 µg Plasmid-DNA und 8 µg Helferplasmid mit *Opi-MEM^{™} I Reduced Serum Medium* sowie *ExpiFectamine^{™} 293 Reagent* vermengt und für 5 Minuten inkubiert. Im Anschluss wurden beide Ansätze vermengt und weitere 20 Minuten inkubiert. Danach wurde der Transfektionsansatz tropfenweise zu den Zellen gegeben. Die Zellen wurden für 24 h dynamisch kultiviert. Danach erfolgte die Selektion der positiven Transfektanten mit Selektionsmedien, die 2 µg/mL Puromycin (ThermoFisher; Waltham, USA) enthielten, für 7 Tage bei 37 °C, 125 rpm und 8 % CO₂.

### Beispiel 3

### Magnetische Zellsortierung (MACS)

Positiv transfizierte HEK293T-Zellen wurden mittels MACS-Technologie nach dem Protokoll des Herstellers sortiert (Miltenyi; Bergisch Gladbach, Deutschland). Kurz gesagt wurden die Zellen durch Zentrifugation (300 x g für 8 min bei 4°C) geerntet und in 200 µL MACS-Puffer (PBS/1% BSA; 2 mM EDTA pH 7,4) resuspendiert und mit 1 µg Maus-Anti-Hämagglutinin (HA)-Antikörper pro 1x106 Zellen gefärbt. Die Färbung wurde für 20 min bei 4°C durchgeführt. Nach der Inkubation wurden die Zellen zweimal in 5 mL MACS-Puffer gewaschen und anschließend zentrifugiert (300 x g für 8 min bei 4°C). Das Zellpellet wurde in 200 µL MACS-Puffer mit 10 µL einer Anti-Maus-IgG-Micro-Bead-Suspension resuspendiert und für weitere 20 min bei 4°C inkubiert. Nach zweimaligem Waschen der Zellen in 5 mL MACS-Puffer wurde das Zellpellet in 3 mL MACS-Puffer resuspendiert und auf eine MACS LS-Säule zur magnetischen Zellseparation aufgetragen. Positive Zellen wurden durch Zugabe von 5 mL MACS-Puffer auf die Säule eluiert, nachdem die Säule aus dem Magnetfeld entfernt wurde. Die positiven Zellen wurden zentrifugiert und das Zellpellet wurde in Expi293 Expression Medium, ergänzt mit 2 µg/mL Puromycin, resuspendiert. Die resuspendierten Zellen wurden in 125 mL Kulturflaschen (TPP, Ort) bei 37°C, 125 rpm und 8% CO2 kultiviert.

### Beispiel 4

### Nanobody Library

Für die Herstellung einer Nanobody Library wurden zunächst periphere mononukleäre Zellen (PBMC) aus dem Vollblut von Lamas (*Llama glama*) isoliert. Aus diesen PBMCs wurde die RNA nach dem Standardprotokoll mit Phenol-Chloroform isoliert (RNAPure, VWR). Im Anschluss erfolgte die Erzeugung der komplementären DNS (cDNA) mittels der Reversen Transkriptase. Diese dient als Grundlage für die Amplifikation der VH und VHH Gene (=initiale PCR). In einer zweiten PCR wurde, sofern gewünscht, zwischen den VH und den VHH Gen-Amplifikaten diskriminiert. Im nächsten Schritt erfolgt die Integration der VHs und/oder VHHs in den zuvor linearisierten Zielvektor über homologe Rekombination. Die daraus resultierenden Vektoren stellen die Nanobody Library dar.

### Beispiel 5

### Fluoreszenzmikroskopie positiver Transfektanten

Für die Immunfluoreszenz wurden 3x105 HA-AP-EGF-R+ HEK293T-Zellen auf mit Poly-L-Lysin beschichteten Glasobjektträgern in 6-WellPlatten ausgesät und in DMEM-Vollmedium, ergänzt mit 2 µg/mL Puromycin, bei 37°C und 8% CO2 über Nacht inkubiert. Die Zellen wurden dann mit sterilem PBS gewaschen und mit 4% Paraformaldehyd in PBS für 20 min bei RT fixiert. Nach der Fixierung wurden die Objektträger mit sterilem PBS gewaschen und mit PBS/1% BSA (20 min bei RT) getränkt, um unspezifische Bindungsereignisse zu vermeiden. Phycoerythrin (PE)-konjugiertes Streptavidindient dem Nachweis des biotinylieren Oberflächenrezeptors. Dafür wurden 2 µg des PE-Streptavidinkonjugates (eBioScience 12-4317; 1E6 Zellen) in PBS/1% BSA verdünnt und für 20 min inkubiert. Danach wurde ein Waschschritt mit PBS/1% BSA durchgeführt. Für die fluoreszenzmikroskopische Analyse wurden die Objektträger noch einmal mit sterilem PBS und einmal mit destilliertem Wasser gewaschen, bevor sie in TissueTek montiert wurden. Die mikroskopischen Aufnahmen wurden mit dem LSM800 (Zeiss; Oberkochen, Deutschland)

### Beispiel 6

### Durchflusszytometrische Analyse

Für die durchflusszytometrischen Analysen wurden die Zellen in FACS-Puffer (PBS mit 0,5 % BSA und 0,01 % Natriumazid) zwei Mal gewaschen. Im Anschluss wurde der biotinylierte Oberflächenrezeptor durch ein Phycoerythrin (PE)-konjugiertes Streptavidin nachgewiesen. Dafür wurden 1 µg des PE-Streptavidinkonjugates (eBioScience 12-4317; 1E6 Zellen) in FACS-Puffer verdünnt und für 20 min inkubiert. Durch den nachfolgenden Waschschritt wurde überschüssiges PE-Streptavidinkonjugat entfernt. Die Zellen wurde in FACS-Puffer erneut aufgenommen und wurden direkt im Anschluss im Durchflusszytometer (BD FACSAria^{™}

## Patentansprüche

1. Verfahren zur Selektion oder zum Screenen ein oder mehrerer Antikörper aus ein oder mehreren Antikörperbibliotheken, wobei mindestens einem nach seiner Transformation stabil in das Genom integrierte Polynukleotide in einem Expressionsvektor in einer Wirtszelle verwendet wird, wobei der Expressionsvektor mindestens aufweist:
i.) Polynukleotide kodierend für mindestens eine Antikörperbibliothek,
ii.) Polynukleotide kodierend für ein Oberflächenprotein enthaltend eine Ligationspeptidsequenz und / oder mindestens ein Epitop für mindestens einen Antikörper aus einer oder mehreren Antikörperbibliotheken,
wobei
a.) die Antikörper der Antikörperbibliothek aus der Wirtszelle in das Kulturmedium sezernieren und an mindestens ein zugegebenes Antigen binden, welche einen Adapter aufweist, wobei der Adapter an die Ligationspeptidsequenz aus ii.) bindet,
und /oder
b.) die Antikörper der Antikörperbibliothek aus der Wirtszelle in das Kulturmedium sezernieren und an mindestens ein zugegebenes Antigen binden, wobei der sezernierte Antikörper an mindestens ein Epitop aus ii.) bindet.

2. Verfahren zur Selektion oder Screenen ein oder mehrerer Antikörper aus ein oder mehreren Antikörperbibliotheken nach Anspruch 1, wobei eine Markierung des gebundenen Antigens aus a.) oder b.) und / oder des gebundenen Antikörpers aus a.) oder b.) erfolgt, insbesondere mit einem gelabelten Antikörper oder magnetischen Bead und mit einem flow sorter (Fluss-Sortierer) oder FACS (fluorescence-activated cell sorting) sortiert und isoliert werden.

3. Verfahren zur Selektion oder Screenen ein oder mehrerer Antikörper aus ein oder mehreren Antikörperbibliotheken nach einem der vorstehenden Ansprüche, wobei die Antikörperbibliothek mindestens eine variable Region der leichten und/oder der schweren Kette und mindestens eine konstante Region aufweisen, insbesondere mindestens einen humanen, murinen oder kameliden Fc-Teil, und mindestens eine Protein-Leader Sequenz.

4. Verfahren zur Selektion oder Screenen ein oder mehrerer Antikörper aus ein oder mehreren Antikörperbibliotheken nach einem der vorstehenden Ansprüche, wobei die Antikörperbibliothek ausgewählt ist aus der Gruppe kombinatorische oder mutagenisierte Antikörperbibliothek insbesondere naive, immune, nicht-immune und semisynthetische Antikörperbibliothek.

5. Verfahren zur Selektion oder Screenen ein oder mehrerer Antikörper aus ein oder mehreren Antikörperbibliotheken nach einem der vorstehenden Ansprüche, wobei die Wirtszelle eine Säugetierzelle ist, insbesondere HEK293-Zellen, CHO-Zellen.

6. Verfahren zur Selektion oder Screenen ein oder mehrerer Antikörper aus ein oder mehreren Antikörperbibliotheken nach einem der vorstehenden Ansprüche, wobei die Ligationspeptidsequenz ein Biotinylierungspeptid ist ausgewählt aus der Gruppe
**1-L**XX**I**XXXX**K**X XXX-13 (SEQ ID No. 3) mit
Aminosäure 2 N, C oder beliebig oder entfernt sein kann,
Aminosäure 3 beliebig, jedoch ausgenommen L, V, I, W, F, Y sein kann,
Aminosäure 5 F oder L sein kann,
Aminosäure 6 E oder D sein kann,
Aminosäure 7 A, G, S, T sein kann,
Aminosäure 8 Q oder M sein kann,
Aminosäure 10 I, M, V sein kann,
Aminosäure 11 E, L, V, Y, I sein kann,
Aminosäure 12 W, Y, V, F, L, I sein kann,
Aminosäure 13 R, H oder beliebig, jedoch ausgenommen D, E oder das Biotinylierungspeptid ist ausgewählt aus der Gruppe GLNDIFEAQKIEWHE (SEQ ID No. 1) oder LNDIFEAQKIEWH (SEQ ID No. 2) und der Adapter ist ausgewählt aus Avidin, Streptavidin, Neutravidin.

7. Verfahren zur Selektion oder Screenen ein oder mehrerer Antikörper aus ein oder mehreren Antikörperbibliotheken nach einem der vorstehenden Ansprüche, wobei der Expressionsvektor Polynukleotide aufweist ausgewählt aus der Gruppe kodierend für mindestens eine Biotin-Proteinligase (BirA), Signalsequenz, HA-Tag (SEQ ID No. 5, SEQ ID No. 6), DNA-Biotinylierungssequenz (SEQ ID No. 7), EGFR-Signalsequenz (SEQ ID No. 8, SEQ ID No. 9), EGFR-Sequenz (SEQ ID No. 10, SEQ ID No. 11 inkl. Transmenbrandomäne), IRES (SEQ ID No. 12), birA-Sequenz (SEQ ID No. 13 inkl. Retentionssignal).

8. Verfahren zur Selektion oder Screenen ein oder mehrerer Antikörper aus ein oder mehreren Antikörperbibliotheken nach einem der vorstehenden Ansprüche, wobei die Zugabe der mehreren und verschiedenen Antigene in der Kälte erfolgt und anschließend in der Wärme die Antikörper der Antikörperbibliothek aus der Wirtszelle in das Kulturmedium sezernieren.

9. Verfahren zur Selektion oder Screenen ein oder mehrerer Antikörper aus ein oder mehreren Antikörperbibliotheken nach einem der vorstehenden Ansprüche, wobei zusätzlich Blockierungsmittel nach Zugabe des mindestens einen Antigens in das Kulturmedium hinzugegeben werden.

10. Expressionsvektor aufweisend
i.) Polynukleotide kodierend für mindestens eine Antikörperbibliothek,
ii.) Polynukleotide kodierend für ein Oberflächenprotein enthaltend eine Ligationspeptidsequenz und / oder mindestens ein Epitop für mindestens einen Antikörper aus einer oder mehreren Antikörperbibliotheken.

11. Säugetierzelle umfassend einen Expressionsvektor nach Anspruch 10.

12. Verwendung von mindestens einem oder mehr als einem Antigen zur Selektion oder zum Screenen ein oder mehrerer Antikörper aus ein oder mehreren Antikörperbibliotheken, wobei mindestens einem nach seiner Transformation stabil in das Genom integrierte Polynukleotide in einem Expressionsvektor in einer Wirtszelle verwendet wird, wobei der Expressionsvektor mindestens aufweist:
i.) Polynukleotide kodierend für mindestens eine Antikörperbibliothek,
ii.) Polynukleotide kodierend für ein Oberflächenprotein enthaltend eine Ligationspeptidsequenz und / oder mindestens ein Epitop für mindestens einen Antikörper aus einer oder mehreren Antikörperbibliotheken,
wobei
a.) die Antikörper der Antikörperbibliothek aus der Wirtszelle in das Kulturmedium sezernieren und an mindestens ein zugegebenes Antigen binden, welche einen Adapter aufweist, wobei der Adapter an die Ligationspeptidsequenz aus ii.) bindet, und /oder
b.) die Antikörper der Antikörperbibliothek aus der Wirtszelle in das Kulturmedium sezernieren und an mindestens ein zugegebenes Antigen binden, wobei der sezernierte Antikörper an mindestens ein Epitop aus ii.) bindet.
